# EUROPEAN PATENT APPLICATION

(11) **EP 3 845 067 A1**
(43) Date of publication of application: **07.07.2021**
(21) Application number: 20150182.2
(22) Date of filing: 03.01.2020
(51) Int. Cl.: A01N 47/46, A61K 31/26, A61P 31/10, A01P 3/00

(54) **FUNGICIDES TO PREVENT AND CONTROL FUNGAL PATHOGENS**

(71) Applicant: AgroSustain SA, 1260 Nyon (CH)
(72) Inventor: DUBEY, Olga, 1028 Préverenges (CH); DUBEY, Sylvain, 1028 Préverenges (CH); Gindro, Katia, 1260 Nyon (CH); Schnee, Sylvain, 1260 Nyon (CH)
(74) Representative: KATZAROV S.A.

(57) **Abstract**

The invention relates to the field of biological fungicides with a broad range of antifungal activity coming from plant extracts from the order of Brassicales or molecules revealing similar chemical structure. In particular, Applicants provided an usage of a combination of sulfonyl and sulfinyl containing aliphatic glucosinolates, their by-products and synthetic analogues as efficient antifungal compounds with broad spectrum of activity.

## Description

### FIELD OF THE INVENTION

The invention relates to the field of biological fungicides with a broad range of antifungal activity coming from plant extracts from the order of Brassicales or molecules revealing a similar chemical structure. In particular, Applicants surprisingly provided a new usage of a combination of two sulfonyl and sulfinyl aliphatic glucosinolates, as well as their by-products as efficient antifungal compounds with broad spectrum of activity.

### BACKGROUND OF THE INVENTION

Human population is increasing each year and is about to reach 8.6 billion by 2030. To maintain high levels of food production, farmers have to use external treatments, such as: 1) **chemical pesticides** that have high efficiency, accessible costs, but reveal negative impact on the environment and human health; 2) **biological pesticides** having no harmful effect on environment, but showing low efficiency (less than 60%, compared to existing chemical pesticide) and high costs. That makes biological pesticides not accessible for many countries and opens possibilities to develop and bring to market novel organic treatments that reveal high efficiency and accessible costs and are environmentally friendly.
In the last decades, some biological approaches were developed to prevent *B. cinerea* in the field, e.g. the application of *Bacillus subtilis* and *Trichoderma harzanium,* but they are poorly used in farming due to their low efficiency.
In western European agriculture, the commonly used bio-preventive fungicides are copper and sulphur. These fungicides are costly to apply due to the need to reapply after each precipitation. In addition, high concentrations of these metals in soil have negative impacts on the environment.
As a consequence, it is crucial to provide an alternative to these techniques, by being more respectful towards the environment, as well as a highly efficient preventive treatment against fungal pathogens.

Plant fungal pathogens are one of the agronomical threats that leads to severe food losses yearly. The efficiency of fungal pathogens is caused by their easy dispersal in nature, rapid attachment on the host surface and fast germ tube development that promotes penetration in plants. Plants, on the other hand, have developed several defence mechanisms against fungal pathogens e.g. necrotrophs: a) prevention of pathogen penetration; b) increased levels of reactive oxygen species; c) induction of defence hormones, such as jasmonate, ethylene, salicylic and abscisic acid. Additionally, some plants are synthetizing fungitoxic compounds that prevent fungal development on plant surface and stop disease formation. Identification of plant compounds with strong antifungal activity can lead to development of novel biological fungicides that can, potentially, replace currently existing chemical treatments.

Order of *Brassicales* consists of economically important plants that are broadly distributed and used as food source. This group of plants was shown to have a unique set of secondary metabolites - glucosinolates. In the last decades, glucosinolates were shown to have anti-cancerous, anti-inflammatory and insecticidal properties.

For example JPH11139949 A (OGAWA KORYO CO LTD) discloses how to obtain an antibacterial-antifungal agent free from strong irritating odour, having a high threshold, low in volatility and having an excellent antibacterial-antifungal activity by compounding a specific [omega]-alkenyl isothiocyanate compound or a specific [omega]-alkylthioalkyl isothiocyanate compound. An [omega]-alkenyl isothiocyanate compound having the formula: CH2 CH(CH2)m NCS [(m)=2-10] (e.g. 3-butenyl isothiocyanate) or an [omega]-alkylthioalkyl isothiocyanate compound having the formula: RS(CH2)n2 NCS [(n)=1-10; R is a 1-4C alkyl] (e.g. methylthiomethyl isothiocyanate) is compounded in a food in an amount of 0.01-100 ppm, preferably 1-50 ppm or in an oral hygienic agent in a concentration of about 0.01-100 ppm.

Similarly, JPH11246319 A (OGAWA KORYO CO LTD) discloses how to obtain an antimicrobial and antifungal agent that can largely alleviate the irritating smell and can be applied to various kinds of beverage and food by using a specific [omega]-alkylsulfinylalkykl isothiocyanate as an active ingredient. This antimicrobial and antifungal agent comprises an [omega]- alkylsulfinylalkyl isothiocyanate represented by the formula: R-S(O)-(CH2)n -NCS (n is 1-10; R is a 1-4C alkyl). In an embodiment, this isothiocyanate is 3-methylsulfinylpropyl isothiocyanate, 6-methylsulfinyl-propyl isothiocyanate or the like. The compound in formula I where n is <=7 is included in the flavour components of horse radish, but its content is low. The compound of the formula is prepared by oxidizing [omega]-methylthioalkyl isothiocyanate of the formula: CH3 S-(CH2)n -NCS with a peroxide.

JP2000086414 A (KINJIRUSHI WASABI KK) discloses how to obtain an antimicrobial agent containing an aromatic component of horseradish as an active ingredient, esp. showing an antibacterial spectrum broadly ranging from fungi to bacteria, and exerting a strong bacteriostatic and antibacterial effect even with an infinitesimal content of the aromatic component. The agent comprises, as an active ingredient, an aromatic component of horseradish of n-methylsulfonylalkylisothiocyanate and one or more aromatic components of horseradish selected from the group consisting of n-methylthioalkyl isothiocyanate, n-methylsulfinylalkyl isothiocyanate, and allyl isothiocyanate. The agent comprises, as an active ingredient, aromatic components of horseradish of both n-methylthioalkyl isothiocyanate and n- methylsulfinylalkyl isothiocyanate.

K. GILLIVER "The Inhibitory Action of Antibiotics on Plant Pathogenic Bacteria and Fungi", ANNALS OF BOTANY., vol. 10, no. 3, 1 July 1946 (1946-07-01), pages 271-282, XP55512767, GB ISSN: 0305-7364, DOI: 10.1093/oxfordjournals.aob.a083136; reports that example of antagonism between soil micro-organisms and plant pathogens have been recognized for many years; and in some instances biological methods of disease control have been elaborated. Specific antibiotic substances have been isolated from culture filtrates of fungi, bacteria and Actinomycetes, and some of them have been shown to have an antagonistic action against causal organisms of plant diseases. In particular, the antifungal activity of cheiroline is disclosed.

In T Sotelo et al. "In vitro activity of glucosinolates and their degradation products against Brassica-Pathogenic bacteria and fungi", Applied and Environmental Microbiology, 1 January 2015 (2015-01-01), pages 432-440, XP55512754, DOI: 10.1128/AEM.03142-14, the objective of the work was to evaluate the biocide effects of 17 Glucosinolates (GSLs) and glucosinolate hydrolysis products (GHPs) and of leaf methanolic extracts of different GSL-enriched Brassica crops on suppressing in vitro growth of two bacterial (Xanthomonas campestris pv. campestris and Pseudomonas syringae pv. maculicola) and two fungal (Alternaria brassicaeand, Sclerotinia scletoriorum) Brassica pathogens. GSLs, GHPs, and methanolic leaf extracts inhibited the development of the pathogens tested compared to the control, and the effect was dose dependent. In particular, this document discloses the antifungal activity of sulorafane.

L DROBNICA et al. "Antifungal activity of Isothiocyanates and related compounds", APPLIED MICROBIOLOGY, vol. 15, no. 4, 1967, pages 701-709, presents the results of a study on the antifungal activity of isothiocyanates-derivatives of biphenyl (group "A"), of stilbene ("B"), of azobenzene and benzeneazonaphthalene ("C"), of naphthalene ("D"), and of further polycondensed aromatic hydrocarbons ("E"). From a total of 48 investigated compounds, antifungal activity was observed only in A and D group compounds. B, C, and E group derivatives are extremely insoluble in water, and the molecules are very large; as a result, they probably cannot pass into spores or mycelium of fungi. It was suggested that the - NCS group cannot manifest its reactivity. In particular, this document discloses the antifungal activity of of glucocheirolin, glucoerysolin and glucoberteroin.

DE 17 93 450 A1 (PHILIPS NV) relates to fungicidal compositions which contain certain thiocyanates, i.e. sulphinyl or sulphonyl-methylene rhodanides, and to methods for preparing and protecting plants against infestation by moulds.

However, there is still a need to provide natural molecules, being respectful towards the environment, but revealing a stronger fungitoxic effect and being active on a broader range of fungal pathogens.

### BRIEF DESCRIPTION OF THE INVENTION

In the present invention, Applicants have identified a combination of two sulfonyl and sulfinyl containing aliphatic glucosinolates namely 1-Isothiocyanato-8-(methylsulfonyl)-octane (8MSOOH) and 1-Isothiocyanato-8-(methylsulfinyl)-octane (8MSOH) and their by-products revealing at a particular concentration a strong fungitoxic effect on a broad range of fungal pathogens. This combination of products can be used as a new line of biological fungicides.

One of the objects of the present invention is to provide a use of a composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and wherein 1-Isothiocyanato-8-(methylsulfonyl)-octane (8MSOOH) represents between 0.5-7% in concentration of said combination of said two active compounds, in the prevention or treatment of fungal pathogens in plants.

Another object of the present invention is to provide a composition comprising the combination of the compounds of the invention for use in a method for preventing or treating mycosis in human or animal patients.

A further object of the invention is to provide a fungicide composition comprising the combination of the compounds of the invention.

The present invention also relates to a food, a drink or an oral hygiene agent containing the above-mentioned antifungal agent, namely the fungicide composition.

Other objects and advantages of the invention will become apparent to those skilled in the art from a review of the ensuing detailed description, which proceeds with reference to the following illustrative drawings, and the attendant claims.

### BRIEF DESCRIPTION OF THE FIGURES

**Figure 1****:** represents the ED50 (in µM) obtained for the two molecules alone (8MSOH and 8MSOOH) and for the combinations of them for three fungal pathogens (*A. radicina, F. graminearum,* and *P. cucumerina*)*.* A star is indicating the lowest ED50 of the tested ratios.
**Figure 2****:** represents by-products of the mixture of 1-Isothiocyanato-8-(methylsulfonyl)-octane (8MSOOH) and 1-Isothiocyanato-8-(methylsulfinyl)-octane (8MSOH).

### DETAILED DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control.
Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

As used in the specification and claims, the singular forms "a", "an" and "the" include plural references unless the context clearly dictates otherwise.

The presence of broadening words and phrases such as "one or more," "at least," "but not limited to" or other like phrases in some instances shall not be read to mean that the narrower case is intended or required in instances where such broadening phrases may be absent.

As used herein the terms "subject" or "patient" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject with a disease or disorder. However, in other embodiments, the subject can be a normal subject. The term does not denote a particular age or sex. Thus, adult and new-born subjects, whether male or female, are intended to be covered.

The term "an effective amount" refers to an amount necessary to obtain a physiological effect. The physiological effect may be achieved by one application dose or by repeated applications. The dosage administered may, of course, vary depending upon known factors, such as the physiological characteristics of the particular composition; the age, health and weight of the subject; the nature and extent of the symptoms; the kind of concurrent treatment; the frequency of treatment; and the effect desired and can be adjusted by a person skilled in the art.

A "fungus" is a eukaryote that digests food externally and absorbs nutrients directly through its cell walls. Most fungi reproduce by spores and have a body (thallus) composed of microscopic tubular cells called hyphae. Fungi are heterotrophs and, like animals, obtain their carbon and energy from other organisms. Some fungi obtain their nutrients from a living host (plant or animal) and are called biotrophs; others obtain their nutrients from dead plants or animals and are called saprotrophs (saprophytes, saprobes). Some fungi infect a living host, but kill host cells in order to obtain their nutrients; these are called necrotrophs.

"Pathogenic fungi" also referred herein as "fungal pathogens" are fungi that cause disease in plants, humans or other organisms. Approximately 300 fungi are known to be pathogenic to humans. The study of fungi pathogenic to humans is called "medical mycology". Although fungi are eukaryotic, many pathogenic fungi are microorganisms. The study of fungi and other organisms pathogenic to plants is called plant pathology.
There are thousands of species of plant pathogenic fungi that collectively are responsible for 70% of all known plant diseases. Plant pathogenic fungi are parasites, but not all plant parasitic fungi are pathogens. Plant parasitic fungi obtain nutrients from a living plant host, but the plant host doesn't necessarily exhibit any symptoms. Plant pathogenic fungi are parasites and cause diseases characterized by symptoms.

"Fungicides" are biocidal chemical compounds or biological organisms used to kill parasitic fungi or their spores (defined herein as fungitoxic). A fungistatic inhibits their growth. Fungi can cause serious damages in agriculture, resulting in critical losses of yield, quality, and profit. Fungicides are used both in agriculture and medicine to fight fungal infections in animals or humans. Chemicals used to control oomycetes, which are not fungi, are also referred to as fungicides, as oomycetes use the same mechanisms as fungi to infect plants. Fungicides can either be contact, translaminar or systemic. Contact fungicides are not taken up into the plant tissue and protect only the plant where the spray is deposited. Translaminar fungicides redistribute the fungicide from the upper, sprayed leaf surface to the lower, unsprayed surface. Systemic fungicides are taken up and redistributed through the xylem vessels. Few fungicides move to all parts of a plant. Some are locally systemic, and some move upwardly.

"Fungistatics" are anti-fungal agents that inhibit the growth of fungus (without killing the fungus). The term fungistatic may be used as both a noun and an adjective. Fungistatics have applications in agriculture, the food industry, the paint industry, and medicine.

One of the objects of the present invention is to provide a use of a composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and wherein 1-Isothiocyanato-8-(methylsulfonyl)-octane (8MSOOH) represents between 0.5-7% in concentration of said combination of said two active compounds, in the prevention or treatment of fungal pathogens in plants.

Preferably, the 1-Isothiocyanato-8-(methylsulfonyl)-octane (8MSOOH) represents between 1-4% in concentration of the combination of said two active compounds, more preferably said 1-Isothiocyanato-8-(methylsulfonyl)-octane (8MSOOH) represents between 1-2% in concentration of the combination of said two active compounds.
However in particular embodiments, the lower concentration of 1-Isothiocyanato-8-(methylsulfonyl)-octane (8MSOOH) in the combination of said two active compounds can be substantially below 0.5% in concentration, being for example 0.4% or 0.3% or even 0.2%.

As used herein the term "concentration" refers to a molar concentration, which is the concentration of a substance expressed in terms of molarity. Molar concentration (also called molarity, amount concentration or substance concentration) is a measure of the concentration of a chemical species, in particular of a solute in a solution, in terms of amount of substance per unit volume of solution. The unit for molarity is the number of moles per litre or mol/L.

The composition of the invention is fungitoxic and/or fungistatic in plants and can be applied to plant cultures in the field or for in vitro implementation thereof.

It is usually accepted that "synergy" occurs when the combined action of two or more agents is greater than the sum of their individual effects. In other words, synergy is said to occur when the combined action of two or more agents is greater than could have been predicted based on the performance of the agents when used alone.

"Isothiocyanate" is the chemical group -N=C=S, formed by substituting the oxygen in the isocyanate group with a sulphur. Many natural isothiocyanates from plants are produced by enzymatic conversion of metabolites called glucosinolates. These natural isothiocyanates, such as allyl isothiocyanate, are also known as mustard oils. An artificial isothiocyanate, phenyl isothiocyanate, is used for amino acid sequencing in the Edman degradation.

Double bonds in principle can have E- or Z-configuration. The compounds of this invention may therefore exist as isomeric mixtures or single isomers. If not specified both isomeric forms are intended. Where a compound of the invention contains one chiral centre, the compound can be provided as a single isomer (R or S) or as a mixture of isomers, for example a racemic mixture. Where a compound of the invention contains more than one chiral centre, the compound can be provided as an enantiomerically pure diastereoisomer or as a mixture of diastereoisomers.

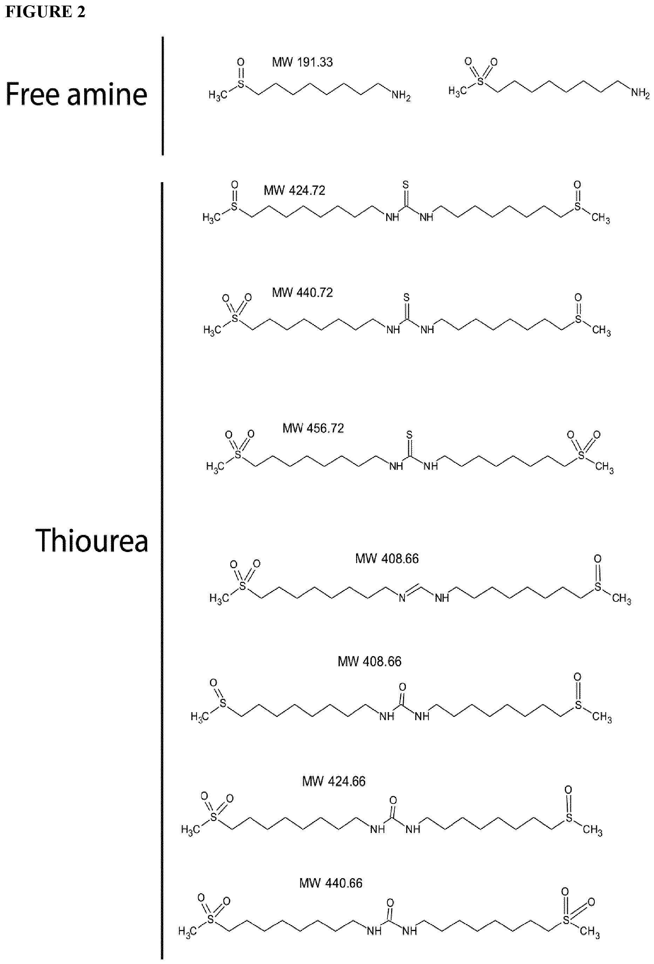

"By-products" refer to compounds that are formed when a reaction goes to completion. By-products are secondary products derived from a production process, biological process or chemical reaction.
By-products of 1-Isothiocyanato-8-(methylsulfonyl)-octane (8MSOOH) and of 1-Isothiocyanato-8-(methylsulfinyl)-octane (8MSOH) consist in free amines and thioureas.
The by-products of the free amines are 8-(Methylsulfinyl)octylamine and 8-(Methylsulfonyl)octylamine. By-products of thioureas are given in figure 2.

The identified compounds of the invention present several advantages, they reveal fungitoxic and/or fungistatic activity against environmental, plant, storage and medical fungal pathogens. Besides, the identified compounds of the invention do not reveal phytotoxicity, are stable in the light and can be freely applied on plants.

The composition used in the present invention has been shown to extend shelf-life by a minimum of one week for fruits, vegetables and cut flowers infected by fungal pathogens in storage facilities. Compounds used (i.e. mixture) were shown to be not toxic for insects and humans. The composition of the invention is easily applicable with a specific impact on the ripening perishable food and no extra installation cost is required. The composition of the invention is of interest to storage companies (i.e. reducing costs in packaging), the timber industry, gardeners and farmers.

Thus, the composition of the invention is to be used as a fungitoxic and/or as a fungistatic agent in plants. The composition of the invention to be used as a fungicide has shown a large efficacy in treating various plants or plant families (hosts). Indeed, the composition of the invention can be used in treating more than 1400 species of agronomical important crops or plants, including order of Solanales, Rosales, Vitales, Poales etc.
The composition of the invention may be used with any part of a plant during any part of its life cycle, including but not limited to seeds, seedlings, plant cells, plants, or flowers.

According to the invention, all plants and plant parts can be treated. By "plants" is meant all plants and plant populations such as desirable and undesirable wild plants, cultivars and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods. By plant parts is meant all above ground and below ground parts and organs of plants such as shoot, leaf, blossom and root, whereby for example leaves, needles, stems, branches, blossoms, fruiting bodies, fruits and seed as well as roots, corms and rhizomes are listed. Crops and vegetative and generative propagating material, for example cuttings, corms, rhizomes, runners and seeds also belong to plant parts.

Among the plants that can be protected by the composition of the invention, mention may be made of major field crops like corn, soybean, cotton, Brassica oilseeds such as *Brassica napus* (e.g. canola), *Brassica rapa, B. juncea* (e.g. mustard) and *Brassica carinata,* rice, wheat, sugarbeet, sugarcane, oats, rye, barley, millet, triticale, flax, vine and various fruits and vegetables of various botanical taxa such as Rosaceae sp. (for instance pip fruit such as apples and pears, but also stone fruit such as apricots, cherries, almonds and peaches, berry fruits such as strawberries), Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp. (for instance banana trees and plantings), Rubiaceae sp. (for instance coffee), Theaceae sp., Sterculiceae sp., Rutaceae sp. (for instance lemons, oranges and grapefruit); Solanaceae sp. (for instance tomatoes, potatoes, peppers, eggplant), Liliaceae sp., Compositiae sp. (for instance lettuce, artichoke and chicory - including root chicory, endive or common chicory), Umbelliferae sp. (for instance carrot, parsley, celery and celeriac), Cucurbitaceae sp. (for instance cucumber - including pickling cucumber, squash, watermelon, gourds and melons), Alliaceae sp. (for instance onions and leek), Cruciferae sp. (for instance white cabbage, red cabbage, broccoli, cauliflower, brussel sprouts, pak choi, kohlrabi, radish, horseradish, cress, Chinese cabbage), Leguminosae sp. (for instance peanuts, peas and beans beans - such as climbing beans and broad beans), Chenopodiaceae sp. (for instance mangold, spinach beet, spinach, beetroots), Malvaceae (for instance okra), Asparagaceae (for instance asparagus); horticultural and forest crops; ornamental plants and flowers including cut flowers; grass i.e. golf fields, turf, as well as genetically modified homologues of these crops.

For example, the composition of the present invention can be used for controlling common fungal diseases such as powdery mildew, rust, downy mildew, and anthracnose on field crops, fruit trees and vegetables.
In addition, the composition of the invention can be used for the treatment of resistant diseases, mainly for the control of wheat powdery mildew, rice blast, rice smut, melon powdery mildew, tomato powdery mildew, apple rust, watermelon Anthracnose and flower powdery mildew. Besides the composition has very good control effects against cucumber downy mildew, grape downy mildew, scab, anthrax, and spotted defoliation.

In a particular embodiment of the invention, the composition of the invention is to be used in the treatment or prevention of tree diseases, caused by fungal pathogens e.g. panama disease of banana, ash dieback.

Besides, the composition of the invention can be used directly in the field in plant cultures but also *in vitro* for example for implementation in plant cultures.

Surprisingly, the composition according to the present invention to be used as a fungicide has been found extremely active on more than 43 fungal pathogens (see list below), from 4 different phylum, 8 different classes and 14 different orders:

| **Species** | **Phylum** | **Class** | **Order** |
|---|---|---|---|
| *Rhizoctonia solani* | Basidiomycota | Agricomycetes | Cantharellales |
| *Thamnidium elegans* | Zygomyceta | Zygomycetes | Mucorales |
| *Phytophthora cactorum* | Oomycota | Oomycetes | Peronosporales |
| *Phytophthora syringae* | Oomycota | Oomycetes | Peronosporales |
| *Phytophthora infestans* | Oomycota | Oomycetes | Peronosporales |
| *Phytophthora agathidicida strain 18407* | Oomycota | Oomycetes | Peronosporales |
| *Phythium sp.* | Oomycota | Oomycetes | Peronosporales |
| *Plasmopara viticola* | Oomycota | Oomycetes | Peronosporales |
| *Aspergillus versicolor* | Ascomycota | Eurotiomycetes | Eurotiales |
| *Aspergillus pseudoglaucus* | Ascomycota | Eurotiomycetes | Eurotiales |
| *Aspergillus sp.* | Ascomycota | Eurotiomycetes | Eurotiales |
| *Penicillium vulpinum* | Ascomycota | Eurotiomycetes | Eurotiales |
| *Penicillium wortmannii* | Ascomycota | Eurotiomycetes | Eurotiales |
| *Trichophyton rubrum* | Ascomycota | Eurotiomycetes | Onygenales |
| *Lasiodiplodia theobromae* | Ascomycota | Dothideomycetes | Botryosphaeriales |
| *Guignardia bidwellii* | Ascomycota | Dothideomycetes | Botryosphaeriales |
| *Alternaria radicina* | Ascomycota | Dothideomycetes | Pleosporales |
| *Phoma exigua* | Ascomycota | Dothideomycetes | Pleosporales |
| *Phoma betae* | Ascomycota | Dothideomycetes | Pleosporales |
| *Helminthosporium solani* | Ascomycota | Dothideomycetes | Pleosporales |
| *Cladosporium langeronii* | Ascomycota | Dothideomycetes | Capnodiales |
| *Cladosporium haloterans* | Ascomycota | Dothideomycetes | Capnodiales |
| *Cladosporium sp.* | Ascomycota | Dothideomycetes | Capnodiales |
| *Fusarium verticillioides* | Ascomycota | Sordariomycetes | Hypocreales |
| *Fusarium gramineum* | Ascomycota | Sordariomycetes | Hypocreales |
| *Fusarium equiseti* | Ascomycota | Sordariomycetes | Hypocreales |
| *Fusarium culmorum* | Ascomycota | Sordariomycetes | Hypocreales |
| *Fusarium avenaceum* | Ascomycota | Sordariomycetes | Hypocreales |
| *Acremonium sp.* | Ascomycota | Sordariomycetes | Hypocreales |
| *Pyricularia oryzae* | Ascomycota | Sordariomycetes | Magnaporthales |
| *Scopulariopsis brevicaulis* | Ascomycota | Sordariomycetes | Microascales |
| *Scopulariopsis fusca* | Ascomycota | Sordariomycetes | Microascales |
| *Colletotrichum acutatum* | Ascomycota | Sordariomycetes | Glomerellales |
| *Colletotrichum coccodes* | Ascomycota | Sordariomycetes | Glomerellales |
| *Colletotrichum gloeosporioides* | Ascomycota | Sordariomycetes | Glomerellales |
| *Plectosphaerella cucumerina* | Ascomycota | Sordariomycetes | Glomerellales |
| *Monilinia laxa* | Ascomycota | Leotiomycetes | Helotiales |
| *Botrytis cinerea* | Ascomycota | Leotiomycetes | Helotiales |
| *Sclerotinia sclerotiorum* | Ascomycota | Leotiomycetes | Helotiales |
| *Gloeosporium album* | Ascomycota | Leotiomycetes | Helotiales |
| *Hymenoscyphus fraxineus* | Ascomycota | Leotiomycetes | Helotiales |
| *Geotrichum candidum* | Ascomycota | Saccharomycetes | Saccaromycetales |
| *Pichia fermentans* | Ascomycota | Saccharomycetes | Saccaromycetales |

In particular, the composition of the present invention has been shown effective against the plant fungal pathogens selected from the phylum comprising Basidiomycota, Zygomyceta, Oomycota or Ascomycota.

For example, the composition of the present invention has been shown particular effective against the plant fungal pathogens: *Botrytis cinerea, Colletotrichum graminicola, Fusarium oxysporum, Sclerotiana sclerotiorum, Verticillium dahlia, Mycospharella gramincola* and *Sphacelotheca reliana.*

In example 2, the combination of 8MSOH/8MSOOH at a ratio of 99/1 proved to be efficient on *in vitro* bioassays of 72 fungal pathogens in a preventive and curative manner.

In another embodiment, the composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and wherein said 1-Isothiocyanato-8-(methylsulfonyl)-octane (8MSOOH) represents between 0.5-7% in concentration of the combination of said two active compounds can be used as an antimycotic in food or drink. The present invention also relates to a food and drink and an oral hygiene agent containing the above-mentioned antifungal agent.

Preferably, the 1-Isothiocyanato-8-(methylsulfonyl)-octane (8MSOOH) represents between 1-4% in concentration of the combination of said two active compounds, more preferably said 1-Isothiocyanato-8-(methylsulfonyl)-octane (8MSOOH) represents between 1-2% in concentration of the combination of said two active compounds.

In particular, the antifungal or antimycotic agent of the present invention can be added to foods or drinks to prevent food spoilage and fungus or contamination by pathogens.
In a particular embodiment of the invention, the composition of the invention can be advantageously used by food distributors or retailers for the shelf life extension of fruits and vegetables by preventing development of plant fungal pathogens. For example, the composition of the invention can extend the shelf life of vegetables, berries, fruits or cut flowers by at least one week.

Preferably, the amount added is in the range of 0.01 to 100 ppm based on the food.
More preferably from 1 to 50 ppm.

Examples of foods and beverages to which the antifungal agent of the present invention is to be blended include fish products, fish sausage, ham, fish meat sausage and ham, surimi products, fish and shellfish dried products, smoked products, salted fish, salted fish, shrimp Semi-solid marine products such as marinated cod roe and the like; livestock meat products such as ham, sausage, bacon and ground meat products; prepared foods such as salads, hamburgers, dumplings, boiled beans, Chinese cabbage, cucumbers, vegetables Pickles such as kimchee, sweet potato, tatami dish, and lotus rosemary; seasoning liquids such as sweet and soup; condiments such as miso and soy sauce; noodles such as raw or boiled buckwheat noodles, udon, pasta, various beverages such as fruit juices, carbonated drinks, tea, milk drinks, lactic acid bacteria beverages, coffee, cocoa, soy milk and the like; fruits and vegetables, Milk powder, fermented milk, butter, cheese, ice cream, dairy products such as cream, caramel, candy, chewing gums, jams, margarine and the like.

In yet another embodiment, the composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and wherein said 1-Isothiocyanato-8-(methylsulfonyl)-octane (8MSOOH) represents between 0.5-7% in concentration of the combination of said two active compounds can be used as a disinfectant in oral hygiene or sanitary articles.

Preferably, the 1-Isothiocyanato-8-(methylsulfonyl)-octane (8MSOOH) represents between 1-4% in concentration of the combination of said two active compounds, more preferably said 1-Isothiocyanato-8-(methylsulfonyl)-octane (8MSOOH) represents between 1-2% in concentration of the combination of said two active compounds.

Preferably, the oral hygiene articles are selected among a dentifrice, a lozenge, a liquid or powdered mouthwash, a coating solution, a halitosis preventing agent, a chewing gum.

The dosage form of the oral hygiene agent to which the antimycotic agent of the present invention is blended may be any of a liquid preparation, a solid preparation, and a semisolid agent, and may be a dentifrice, a lozenge, a liquid or powdered mouthwash, Coating solution, halitosis preventing agent, chewing gum and the like.

Furthermore, the disinfectant of the present invention can be used in various products socalled antifungal goods (goods) such as kitchen utensils such as chopping boards, washing tools such as toothbrushes, stationery such as writing instruments, erasers and notes, clothing such as underwear products such as automobile interior items such as handles and seats; home appliances such as word processors and refrigerators; interior decoration materials such as tatami mats and wallpaper, or any sterilization of surfaces e.g. in the building or hospitals' equipment. That is, the antimycotic agent is kneaded into raw materials at the production stage of the above-mentioned commodity and mixed, or by adding an adjuvant such as a surfactant as necessary with this solvent to a product surface. Apply by applying or spraying as a spray. The liquid formulation or spray formulation can be applied as sanitary sheds, toilet seats, disinfectants for bathroom dails and mildewproofing agents. Furthermore, it can also be applied as a sanitary article such as a disinfecting cleaner or a wet tissue by impregnating the liquid formulated agent with a liquid-absorbent support such as paper or cloth.

Advantageously, the composition of the invention can also be used for the treatment or prevention of microbial infection selected from the list consisting of bacterial, fungal, yeast and / or viral infection.

In accordance with an embodiment of the invention, the bacterial infection comprises a gram positive or gram-negative infection.

In particular, the bacterial or yeast infection is a *Pseudomonas aeruginosa, Escherichia coli, S. aureus, S. epidermis, Klebsiellae pneumoniae, Acinetobacter baumannii, B. subtilis, E. aerogenes, C. freundii, Staphylococcus spp, Streptococcus spp, Enterococcus spp, Proteus spp, Candida spp, Apophysomyces spp, Aspergillus, Mucor spp., Porphymonas gingivalis, Prevotella intermedia, Treponema denticola, Tannerella forsythensis* or *Aggregatibacter actinomycetemcomitans* infections.

It is another object of the invention to provide a composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and wherein said 1-Isothiocyanato-8-(methylsulfonyl)-octane (8MSOOH) represents between 0.5-7% in concentration of the combination of said two active compounds, for use in a method for preventing or treating mycosis in human or animal patients.
Preferably, the composition is a pharmaceutical composition.

Preferably, the 1-Isothiocyanato-8-(methylsulfonyl)-octane (8MSOOH) represents between 1-4% in concentration of the combination of said two active compounds, more preferably said 1-Isothiocyanato-8-(methylsulfonyl)-octane (8MSOOH) represents between 1-2% in concentration of the combination of said two active compounds.

The pharmaceutical composition according to the invention can be used for the preparation of a medicament useful to curatively or preventively treat human or animal fungal diseases such as, for example, mycoses, chytrid infections (Bd; Bs), dermatoses, trichophyton diseases and candidiases or diseases caused by Aspergillus spp., for example Aspergillus fumigatus.

In an embodiment of the invention, the mycosis is due to *Candida albicans* or *Trichophyton rubrum* infections.

In particular, the antifungal agent of the present invention has an excellent antimicrobial activity against *Candida albicans,* which is regarded as one of causative yeast of denture stomatitis, so if it is blended into an oral hygiene agent according to a conventional method, Candida's infection can be effectively prevented and treated.

Besides, the antifungal agent of the present invention can be used in combination with other antimicrobial agents such as alcohol, spice ingredients such as sage and rosemary; organic acids such as citric acid, lactic acid, acetic acid and the like.

It is yet another object of the invention to provide a fungicide composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and wherein said 1-Isothiocyanato-8-(methylsulfonyl)-octane (8MSOOH) represents between 0.5-7% in concentration of the combination of said two active compounds.
However in particular embodiments, the lower concentration of 1-Isothiocyanato-8-(methylsulfonyl)-octane (8MSOOH) in the combination of said two active compounds can be substantially below 0.5% in concentration, being for example 0.4% or 0.3% or even 0.2%.

Preferably, the 1-Isothiocyanato-8-(methylsulfonyl)-octane (8MSOOH) represents between 1-4% in concentration of the combination of said two active compounds, more preferably said 1-Isothiocyanato-8-(methylsulfonyl)-octane (8MSOOH) represents between 1-2% in concentration of the combination of said two active compounds.

In particular, the fungicide composition of the present invention has been shown effective against the plant fungal pathogens selected from the phylum comprising: Basidiomycota, Zygomyceta, Oomycota, Ascomycota.

In example 2, the combination of 8MSOH/8MSOOH at a ratio of 99/1 proved to be efficient on *in vitro* bioassays of 72 fungal pathogens in a preventive and curative manner.

For example, the fungicide composition of the present invention has been shown particularly effective against the plant fungal pathogens: *Botrytis cinerea, Colletotrichum graminicola, Fusarium oxysporum, Sclerotiana sclerotiorum, Verticillium dahlia, Mycospharella gramincola* and *Sphacelotheca reliana.*

In an embodiment of the invention, the fungicide composition of the invention is applied in combination with acceptable carriers or diluents, i.e. in a spray.

The fungicide composition of the invention will often be concentrated formulations that can be diluted in water, or another liquid, for application. In certain embodiments, the fungicide composition can also be formulated into particles or granular formulations that are sprayed or applied without further treatment.

Preferably, carriers or diluents to be used in the present invention are phytologically-acceptable.
As used herein, the term "phytologically-acceptable" formulations refers to compositions, diluents, excipients, and/or carriers that are generally applicable for use with any part of a plant during any part of its life cycle, including but not limited to seeds, seedlings, plant cells, plants, or flowers. The formulations can be prepared according to procedures, methods and formulas that are conventional in the agricultural arts. Following the teachings of the present invention, the person skilled in the agricultural and/or chemical arts can readily prepare a desired composition. Most commonly, the fungicide composition of the present invention can be formulated to be stored, and/or applied, as aqueous or non-aqueous suspensions or emulsions prepared neat or from concentrated formulations of the compositions. Watersoluble, water-suspendable or emulsifiable formulations can also be converted into or formulated as solids (e.g., wettable powders), which can then be diluted into a final formulation. In certain formulations, the fungicide compositions of the present invention can also be provided in growth media, e.g., in vitro media for growth of plant or other types of cells, in laboratory plant growth media, in soil, or for spraying on seeds, seedlings, roots, stems, stalks, leaves, flowers or the entire plant.

These phytologically-acceptable formulations are produced in a known manner, for example by mixing the fungicide composition of the invention with extenders, that is liquid solvents, liquefied gases under pressure, and/or solid carriers, optionally with the use of surfactants, that is emulsifiers and/or dispersants, and/or foam formers. If the extender used is water, it is also possible to use, for example, organic solvents as auxiliary solvents. Essentially, suitable liquid solvents include: aromatics such as xylene, toluene or alkylnaphthalenes, chlorinated aromatics or chlorinated aliphatic hydrocarbons such as chlorobenzenes, chloroethylenes or methylene chloride, aliphatic hydrocarbons such as cyclohexane or paraffins, for example petroleum fractions, alcohols such as butanol or glycol and their ethers and esters, ketones such as acetone, methyl ethyl ketone, methyl isobutyl ketone or cyclohexanone, strongly polar solvents such as dimethylformamide and dimethyl sulphoxide, or else water. Liquefied gaseous extenders or carriers are to be understood as meaning liquids which are gaseous at ambient temperature and under atmospheric pressure, for example aerosol propellants such as butane, propane, nitrogen and carbon dioxide. Suitable solid carriers are: for example ground natural minerals such as kaolins, clays, talc, chalk, quartz, attapulgite, montmorillonite or diatomaceous earth, and ground synthetic minerals such as finely divided silica, alumina and silicates. Suitable solid carriers for granules are: for example crushed and fractionated natural rocks such as calcite, marble, pumice, sepiolite and dolomite, or else synthetic granules of inorganic and organic meals, and granules of organic material such as sawdust, coconut shells, maize cobs and tobacco stalks. Suitable emulsifiers and/or foam formers are: for example, nonionic and anionic emulsifiers, such as polyoxyethylene fatty acid esters, polyoxyethylene fatty alcohol ethers, for example alkylaryl polyglycol ethers, alkylsulphonates, alkyl sulphates, arylsulphonates, or else protein hydrolysates. Suitable dispersants are: for example, lignin-sulphite waste liquors and methylcellulose.

The fungicide composition according to the invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure), gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra-low volume (ULV) liquid, ultra-low volume (ULV) suspension, water dispersible granules or tablets, water dispersible powder for slurry treatment, water soluble granules or tablets, water soluble powder for seed treatment and wettable powder. These compositions include not only compositions that are ready to be applied to the plant or seed to be treated by means of a suitable device, such as a spraying or dusting device, but also concentrated commercial compositions that must be diluted before application to the crop.

In a preferred embodiment of the invention, the composition can be specifically applied on fruits and vegetables in storage facilities with an ultrasonic fogger. The ultrasonic fogger is a device that is using ultrasonic sound waves to break water into very small droplets (<10um) and sprays it into the air as a dense cold fog (i.e. not resulting from boiling water). Examples of ultrasonic foggers and systems are i.e. described in the following U.S. Patents: U.S. Pat. No. 4,042,016; U.S. Pat. No. 4,058,253; U.S. Pat. No. 4,118,945; U.S. Pat. No. 4,564,375; U.S. Pat. No. 4,667,465; U.S. Pat. No. 4,702,074; U.S. Pat. No. 4,731,990; U.S. Pat. No. 4,731,998; U.S. Pat. No. 4,773,846; US No. 5,454,518; US No. 6,854,661. Usually an ultrasonic fogger includes: a generally cylindrical body having an axial bore with an outlet at a front face of the body; a gas supply and a liquid supply coupled to the bore; at least a portion of the front face having a curved convex contour, the front face having a flat central annular region surrounding the outlet of the bore; and a resonator spaced from and opposing the outlet end of the bore. Such devices are commonly used to control humidity level in greenhouses, to deliver nutrients to plant in aeroponic cultures or to create optimal humidity levels in houses.
Applicants demonstrated that this technique can be used to apply products used for the extension of fruits and vegetables freshness in storage facilities e.g. natural fungicides. This technology, permits to efficiently treat fruits and vegetables that are not accessible to the treatments applied by spray or other applications, due to their packaging (i.e. it cannot be easily sprayed directly because fruits and vegetables are stored e.g. in container or because spraying might damage the fruits and vegetables).
Interestingly, Applicants performed efficacy trials on fruits and vegetables in a storage facility of 30m³ using an industrial MHS15 Ultrasonic Humidifiers (http://www.mainlandmart.com/humidify.html), generating 15kg of fog / hour made of droplets of a size comprised between 1-10 µm with average 5 micron. The composition of the invention, represented by powder was added into water container of the fogger in a dosage of 400-600 µM, depending on the tested plant species. Powder was dissolved in the water and applied on the fruits in a form of cold vapor. Applicants, have confirmed equal distribution of the product on all treated crops and easy access to the inaccessible previously parts of the package.

The fungicide composition according to the invention can also be mixed with one or more insecticides, pesticides, attractants, sterilants, bactericides, acaricides, nematicides, other fungicides, growth regulators, herbicides, fertilizers, safeners, semiochemicals, flavour exhauster agents or other compounds with biological activity. The mixtures thus obtained have normally a broadened spectrum of activity. The mixtures with other fungicide compounds are particularly advantageous.

The fungicide composition according to the invention comprising a mixture with a bactericide compound can also be particularly advantageous. Examples of suitable bactericide mixing partners can be selected in the following list: bronopol, dichlorophen, nitrapyrin, nickel dimethyldithiocarbamate, kasugamycin, octhilinone, furancarboxylic acid, oxytetracycline, probenazole, streptomycin, tecloftalam, copper sulfate and other copper preparations.

The dose of the fungicide composition usually applied in the treatment according to the invention is generally and advantageously from 10 to 800 g/ha, preferably from 50 to 300 g/ha for applications in foliar treatment. The dose of fungicide composition applied is generally and advantageously from 2 to 200 g per 100 kg of seed, preferably from 3 to 150 g per 100 kg of seed in the case of seed treatment.

It is clearly understood that the doses indicated herein are given as illustrative examples of the treatment method according to the invention. A person skilled in the art will know how to adapt the application doses, notably according to the nature of the plant or crop to be treated.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes, which come within the meaning and range of equivalency, are intended to be embraced therein.

The foregoing description will be more fully understood with reference to the following Examples. Such Examples, are, however, exemplary of methods of practising the present invention and are not intended to limit the scope of the invention.

### Examples

### Example 1:

### In vitro bioassays of three fungal pathogens

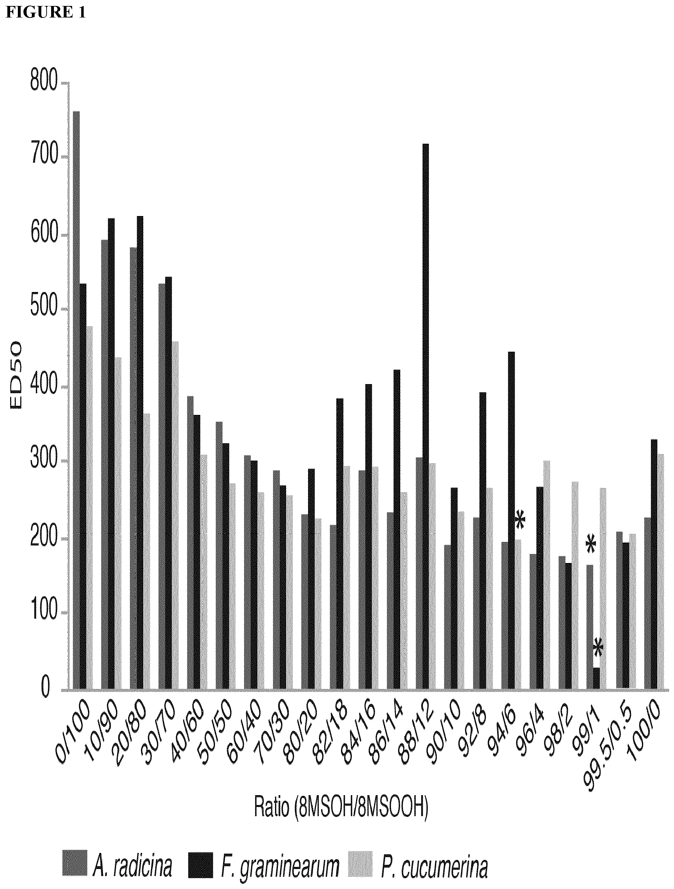

8MSOH and 8MSOOH were obtained from SpiroChem (Basel, Switzerland). Both compounds were dissolved in dimethyl sulfoxide (DMSO) at a stock concentration of 1M. Pathogenic fungal strains used for in vitro bioassays were obtained from the mycotheca of Agroscope (www.mycoscope.bcis.ch) for *Alternaria radicina* and *Fusarium graminearum. Plectosphaerella cucumerina* was isolated from commercial fruits and identified by Sanger sequencing.
Pure cultures of the three species of plant fungal pathogens were inoculated on PDA implemented with different combinations of 8MSOH/8MSOOH mixtures that were preliminary dissolved in PDB and placed in 24-well sterile culture plates (VWR International, Dietikon, Switzerland) to the final concentrations of 10, 75, 150, 250, 375, 500, 700 and 850 µM, respectively. In a first time, bioassays were performed three times (each time in triplicates) for each fungus using different combinations ranging every 10%, i.e. 100/0, 90/10, 80/20, ..., 10/90 and 0/100 for 8MSOH/8MSOOH. In a second time, bioassays were performed in triplicates for each fungus on different combinations ranging every 2% where the effect was optimal in the first bioassays, i.e. 100/0, 99.5/0.5, 99/1, 98/2, 96/4, ..., 82/18 and 80/20 for 8MSOH/8MSOOH. For each bioassay, 2-mm agar plugs of fungal cultures were placed in each well and plates were incubated for 7 days in a phytotron (80% relative humidity, constant temperature of 23°C) under alternating 16 h day and 8 h night cycles. Mycelia growth was measured after 7 days using ImageJ (http://imagej.net/Welcome). EC50 was evaluated as described in Schnee et al., 2013.
Synergistic fungitoxic activities was then estimated as in Chou (2006), and the CompuSyn software (www.combosyn.com) was used to determine the Combination Index (CI) for combinations of two molecules and the presence of synergism (CI<1). The results obtained for each fungal species are summarized in Table 1 and Figure 1. It shows the ED₅₀ (in µM) obtained for the two molecules alone, for the combination of them, and the lower Combination Index (of the tested concentrations: 10 to 850 µM) for the lowest ED50 of the tested combinations.

**Table 1: ED₅₀ (in µM) obtained for the two molecules alone, for the combination of them, and the Combination Index for the lowest ED50 of the tested concentrations (10 to 850 µM).**

| **Ratio (8MSOH/8MSOOH)** | ***A. radicina*** | ***F. graminearum*** | ***P. cucumerina*** |
|---|---|---|---|
| 100/0 | 228 | 331 | 312 |
| 99.5/0.5 | 206 | 192 | 204 |
| 99/1 | **166 (0.22)** | **30 (0.076)** | 267 |
| 98/2 | 177 | 168 | 275 |
| 96/4 | 180 | 268 | 303 |
| 94/6 | 196 | 446 | **199 (0.491)** |
| 92/8 | 228 | 393 | 267 |
| 90/10 | 192 | 267 | 236 |
| 88/12 | 307 | 720 | 300 |
| 86/14 | 235 | 423 | 261 |
| 84/16 | 290 | 404 | 295 |
| 82/18 | 218 | 385 | 296 |
| 80/20 | 232 | 292 | 227 |
| 70/30 | 290 | 270 | 257 |
| 60/40 | 310 | 303 | 261 |
| 50/50 | 354 | 326 | 273 |
| 40/60 | 388 | 363 | 311 |
| 30/70 | 536 | 545 | 460 |
| 20/80 | 584 | 625 | 365 |
| 10/90 | 594 | 622 | 439 |
| 0/100 | 763 | 536 | 480 |

**Conclusions:** The lowest ED50 for *A. radicina, F. graminearum,* and *P. cucumerina* were respectively for the ratio 99/1 (ED50: 166 µM), 99/1 (30 µM) and 94/6 (199 µM). The lowest CI of the lowest ED50 for the three fungal pathogens were respectively 0.22, 0.076 and 0.491, indicating that the combined molecules have strong synergisms and are efficient against the tested fungal species.

### Example 2:

### In vitro bioassays of 72 fungal pathogens

8MSOH and 8MSOOH were obtained from SpiroChem (Basel, Switzerland). Both compounds were dissolved in dimethyl sulfoxide (DMSO) at a stock concentration of 1M. Pathogenic fungal strains used for in vitro bioassays were obtained from the mycotheca of Agroscope (www.mycoscope.bcis.ch) or from commercial fruits and identified by Sanger sequencing.
Pure cultures of the 71 of plant fungal pathogens were inoculated on PDA implemented with one combination of 8MSOH/8MSOOH mixture (99/1) that were preliminary dissolved in PDB and placed in 24-well sterile culture plates (VWR International, Dietikon, Switzerland) to the final concentrations of 10, 75, 150, 250, 375, 500, 700 and 850 µM. For each bioassay, 2-mm agar plugs of fungal cultures (curative test) were placed in each well and plates were incubated for 7 days in a phytotron (80% relative humidity, constant temperature of 23°C) under alternating 16 h day and 8 h night cycles. Similarly, a germination test was performed by applying a concentrated spore solution in each well. Mycelia growth was measured after 7 days using ImageJ (http://imagej.net/Welcome). EC50 was evaluated as described in Schnee et al., 2013.

The list below summarizes the ED50 of the germination and curative tests for 71 fungal pathogens: *Acremonium* sp. (276;420), *Alternaria radicina* (109;166), *Alternaria tenuissima* (<250;1419), *Aspergillus pseudoglaucus* (217;5), *Aspergillus versicolor* (540;1404), *Bionectria ochroleuca* (105;171.57), *Botryosphaeria parva* (<250;<250), *Botrytis cinerea* (156;395), *Cladosporium haloterans* (71;123), *Cladosporium langeronii* (110;190), *Cladosporium* sp. (59;322), *Cladosporium tenuissimum* (304;1113), *Colletotrichum acutatum* (222;211), *Colletotrichum coccodes* (218;534), *Colletotrichum gloeosporioides* (162;376), *Colletotrichum musae* (95;117), *Diaporthe pseudomangiferae* (<250;640), *Fusarium avenaceum* (276;440), *Fusarium avenaceum* (384;707), *Fusarium culmorum* (41;63), *Fusarium gramineum* (47;30), *Fusarium incarnatum* (<250;182), *Fusarium musae* (256;551), *Fusarium oxysporum* (105;272), *Fusarium proliferatum* (245;513), *Fusarium verticillioides* (55;94), *Geotrichum candidum* (>500;515), *Geotrichum candidum* (382;542), *Gloeosporium album* (20;236), *Guignardia bidwellii* (7;9.6'), *Hanseniaspora opuntiae* (278;306), *Hanseniaspora uvarum* (270;310), *Helminthosporium solani* (105;130), *Hymenoscyphus fraxineus* (1;98), *Hyphopichia burtonii* (75;160), *Lasiodiploda pseudotheobromae* (<250;281), *Lasiodiplodia theobromae* (210;190), *Lasiodiplodia theobromae* (105;163), *Lasiodiplodia theobromae* (<250;270), *Monilinia fructigena* (30;43), *Monilinia laxa* (1;1), *Myrothecium* sp. (<50;172), *Nectria pseudotrichia* (101;117), *Nigrospora sphaerica* (<50;129), *Penicillium cellarum* (61;115), *Penicillium crustosum* (274;547), *Penicillium expansum* (267;658), *Penicillium expansum* (301;525), *Penicillium ochrosalmoneum* (112;289), *Penicillium roqueforti* (316;264), *Penicillium solitum* (201;753), *Penicillium solitum* (156;389), *Penicillium solitum* (110;382), *Penicillium vulpinum* (272;93), *Penicillium wortmannii* (107;190), *Pestalotiopsis microspora* (111;722), *Phoma betae* (13;266), *Phoma exigua* (251;543), *Phomopsis* sp. (<250;158), *Phytophthora agathidicida* strain (1;79), *Phytophthora cactorum* (119;75), *Phytophthora infestans* (242;270), *Phytophthora syringae* (36;78), *Pichia fermentans* (218;159), *Pichia kluyveri* (381;402), *Pichia kudriavzevii* (159;323), *Plectosphaerella cucumerina* (158;266), *Rhizoctonia solani* (7;100), *Sclerotinia sclerotiorum* (161;550), *Thamnidium elegans* (248;395), *Tricophyton rubrum* (275;204).

**Conclusions:** The combination of 8MSOH/8MSOOH at a ratio of 99/1 proved to be efficient against a large number of fungal pathogens in a preventive and curative manner.

**List of molecules used in the examples**

| | |
|---|---|
| Molecule abbreviation | IUPAC name |
| 8MSOOH | 1-Isothiocyanato-8-(methylsulfonyl)-octane |
| 8MSOH | 1-Isothiocyanato-8-(methylsulfinyl)-octane |

### REFERENCES

1. Schnee S, Queiroz EF, Voinesco F, Marcourt L, Dubuis P-H, Wolfender J-L, Gindro K. 2013. Vitis vinifera canes, a new source of antifungal compounds against Plasmopora viticola, Erysiphe necator, and Botrytis cinerea. J. Agric. Food Chem. 61(23):5459-67.
2. Chou TC. 2006. Theoretical basis, experimental design, and computerized simulation of synergism and antagonism in drug combination studies. Pharmacol. Rev 58:621-681, 2006.

## Claims

1. Use of a composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and **characterized in that** 1-Isothiocyanato-8-(methylsulfonyl)-octane (8MSOOH) represents between 0.5-7% in concentration of said combination of said two active compounds, in the prevention or treatment of fungal pathogens in plants.

2. The use of the composition of claim 1, **characterized in that**, 1-Isothiocyanato-8-(methylsulfonyl)-octane (8MSOOH) represents between 1-4% in concentration of the combination of said two active compounds.

3. The use of the composition according to any of claims 1-2, **characterized in that** said 1-Isothiocyanato-8-(methylsulfonyl)-octane (8MSOOH) represents between 1-2% in concentration of the combination of said two active compounds.

4. The use of the composition according to any of claims 1-3, **characterized in that** said composition is fungitoxic and/or fungistatic in plants.

5. The use of the composition according to any of claims 1-4, in plant cultures in the field or for in vitro implementation thereof.

6. The use of a composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and **characterized in that** 1-Isothiocyanato-8-(methylsulfonyl)-octane (8MSOOH) represents between 0.5-7% in concentration of the combination of said two active compounds; as an antimycotic in food or drink.

7. The use of the composition according to claim 6, **characterized in that** said 1-Isothiocyanato-8-(methylsulfonyl)-octane (8MSOOH) represents between 1-2% in concentration of the combination of said two active compounds.

8. The use of a composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and **characterized in that** 1-Isothiocyanato-8-(methylsulfonyl)-octane (8MSOOH) represents between 0.5-7% in concentration of the combination of said two active compounds; as a disinfectant in oral hygiene or sanitary articles.

9. The use of the composition according to claim 8, **characterized in that** said 1-Isothiocyanato-8-(methylsulfonyl)-octane (8MSOOH) represents between 1-2% in concentration of the combination of said two active compounds.

10. The use of the disinfectant composition according to any of claims 8-9, wherein the oral hygiene articles are selected among a dentifrice, a lozenge, a liquid or powdered mouthwash, a coating solution, a halitosis preventing agent, a chewing gum.

11. A composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and **characterized in that** 1-Isothiocyanato-8-(methylsulfonyl)-octane (8MSOOH) represents between 0.5-7% in concentration of the combination of said two active compounds, for use in a method for preventing or treating mycosis in human or animal patients.

12. The composition for use according to claim 11, wherein the mycosis is due to *Candida albicans* or *Trichophyton rubrum* infections.

13. A fungicide composition comprising the combination of two active compounds consisting in the mixture of 1-Isothiocyanato-8-(methylsulfonyl)-octane (8MSOOH) and 1-Isothiocyanato-8-(methylsulfinyl)-octane (8MSOH) or their by-products and **characterized in that** 1-Isothiocyanato-8-(methylsulfonyl)-octane (8MSOOH) represents between 0.5-7% in concentration of said combination of said two active compounds.

14. The fungicide composition of claim 11, **characterized in that**, 1-Isothiocyanato-8-(methylsulfonyl)-octane (8MSOOH) represents between 1-4% in concentration of the combination of said two active compounds.

15. The fungicide composition according to any of claims 11-12, **characterized in that** said 1-Isothiocyanato-8-(methylsulfonyl)-octane (8MSOOH) represents between 1-2% in concentration of the combination of said two active compounds.
